# EUROPEAN PATENT SPECIFICATION

(11) **EP 1 386 638 B1**
(45) Date of publication and mention of the grant of the patent: **03.09.2008**
(21) Application number: 03014591.6
(22) Date of filing: 08.07.2003
(51) Int. Cl.: A61N 5/06

(54) **Lighting device**
Beleuchtungseinrichtung
Dispositif d'éclairage

(30) Priority: 02.08.2002 IT TV20020030
(43) Date of publication of application: 04.02.2004
(73) Proprietor: Check up S.p.A., 31013 Codogné, (TV) (IT)
(72) Inventor: Carlet, Michele, 31020 Tarzo (IT)
(74) Representative: Modiano, Micaela Nadia

(56) References cited:
- EP-A- 0 482 680
- WO-A1-95/26217
- JP-A- 62 123 258
- US-A- 5 489 827
- US-A- 6 150 774
- US-B1- 6 350 275

## Description

The present invention relates to a lighting device for the use of color therapy in sanitary fixtures such as for example whirlpool baths, saunas and shower cubicles.

Nowadays, these sanitary fixtures are used increasingly often not only for personal hygiene but also as tools for relaxation of the body and of the nervous system.

Devices are in fact known which associate with the normal functions of a sauna or whirlpool bath the presence of music and/or colored lights.

In particular, the use of knowledge in the field of color therapy has been introduced recently.

Lighting devices for the use of color therapy are in fact currently commercially available which comprise a panel that is associable with a chosen surface and is adapted to support a plurality of monochrome light sources, constituted by electrical components commonly known as LEDs.

The panel supports several sets of LEDs, and each set is characterized by a predefined color and has an approximately uniform arrangement along the entire surface of the panel.

In this manner, proximate to a LED of a given color there are LEDs of a preferably different color.

Five LEDs of different colors, namely green, yellow, blue, red and white, are currently commercially available.

Accordingly, this known type of lighting device can comprise at most five different sets of LEDs, each one associated with one of the colors cited above.

A control unit switches on one or more sets of LEDs, so as to generate light of a single color, if a single set is switched on, or lights of different colors, if two or more sets of LEDs are switched on.

A translucent sheet can be arranged in front of the panel and is suitable to diffuse the light generated by the lit LEDs, so as to mix the color tints emitted by said LEDs.

The color therapy program can provide for a cycle constituted by a plurality of different colors emitted in succession or for a steady light of a preset color chosen among the available colors.

The main drawback of this known type of lighting device is that it can emit light of a preset color, chosen among a very limited number of possible combinations.

Another important drawback is that it is necessary to use a reduced number of LEDs of the same color, since the panel has a limited surface: this does not allow to achieve sufficient brightness whenever a "pure" color, i.e., a color that coincides with the color of one of the sets of LEDs (and therefore the colors green, yellow, blue, red or white) is switched on.

In these cases it is in fact necessary to switch off all the other sets of LEDs, therefore entailing a great reduction in luminous intensity.

Another drawback of the known art is that in order to have a sufficient diffusion of the light emitted by the LEDs it is necessary to use a translucent sheet of considerable thickness.

In any case, it is very difficult to achieve, along the entire surface of the sheet, an effective mixing of the color tints emitted by LEDs of different colors.

The current known art, moreover, entails that the variation of the coloring of the lighting device is achieved by switching on and/or off one or more sets of LEDs.

Accordingly, there is the problem of not being able to achieve a uniform variation of the color tint of the light source, which is an important requirement in color therapy theory: instead the luminous intensity is essentially shifted from some regions of the translucent sheet to other regions depending on the arrangement of each LED.

For example, in order to save on manufacturing costs it is known to provide lighting devices that do not use white-light LEDs, since these LEDs are the most expensive.

To generate white, instead, the green, blue and red sets of LEDs are switched on, and their mixing should indeed generate the color white.

Actually, the less than optimum mixing of these three colors generates, on the outer surface of the sheet, multiple halos of indefinite color, which are very unpleasant for the person who undergoes this type of treatment.

Document US 6 150 774 discloses a multicolored LED lighting method and apparatus that has the features set forth in the preamble of claim 1. A lighting device for sanitary fixtures is also known from document JP 62123258.

The aim of the present invention is to solve the above-noted problems, eliminating the drawbacks of the cited known art, by providing a lighting device that allows optimum use of color therapy, in the field of sanitary fixtures.

Within this aim, an object of the invention is to provide a device that accordingly allows to illuminate a preset surface according to a chosen color tint.

Another object is to provide a device that has a uniform distribution of light along all of said lit surface.

Another object of the present invention is to provide a lighting device that can vary the color tint, optionally in a fully automatic manner, according to a chosen color sequence, chosen from a very wide range of available tints of color.

Another object is to provide a lighting device that can, if required, emit light at a color intensity that is always constant regardless of the color tint chosen.

Another object is to provide a device that is structurally simple and has low manufacturing costs.

This aim and these and other objects that will become better apparent hereinafter are achieved by a lighting device, according to the present invention, that has the features set forth in claim 1.

Further characteristics and advantages of the invention will become better apparent from the following detailed description of a particular embodiment thereof, illustrated by way of non-limitative example in the accompanying drawings, wherein:
Figure 1 is a schematic perspective view of a lighting device;
Figures 2 and 3 are respectively a front view and a side view of a triple-junction LED;
Figure 4 is a front view of the support on which the triple-junction LEDs and the presence detection photocell are installed;
Figure 5 is a block diagram of the operation of the lighting device.

In the embodiments that follow, individual characteristics, given in relation to specific examples, may actually be interchanged with other different characteristics that exist in other embodiments.

Moreover, it is noted that anything found to be already known during the patenting process is understood not to be claimed and to be the subject of a disclaimer.

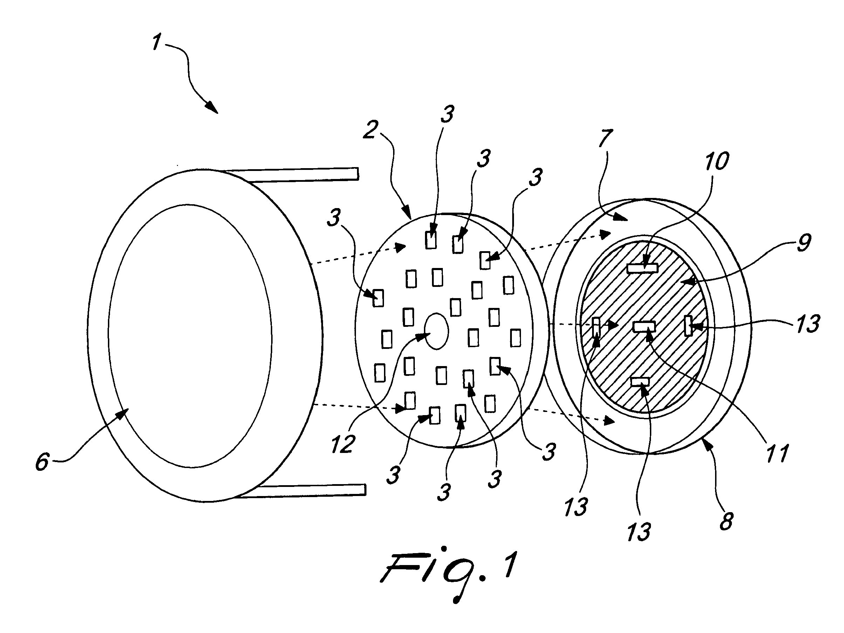

With reference to Figure 1, the reference numeral 1 designates a lighting device that can be used in particular for the use of color therapy in sanitary fixtures such as for example saunas, shower cubicles or whirlpool baths.

The lighting device 1 comprises at least one support, which is advantageously constituted, in this particular exemplifying embodiment of the invention, by a circular plate 2.

The plate 2 acts as a support for a plurality of light sources with modulatable color tint, generally designated by the reference numeral 3.

The light sources 3 are advantageously arranged with an approximately uniform and equidistant distribution on the upper surface of the plate 2, keeping a preset minimum distance between them.

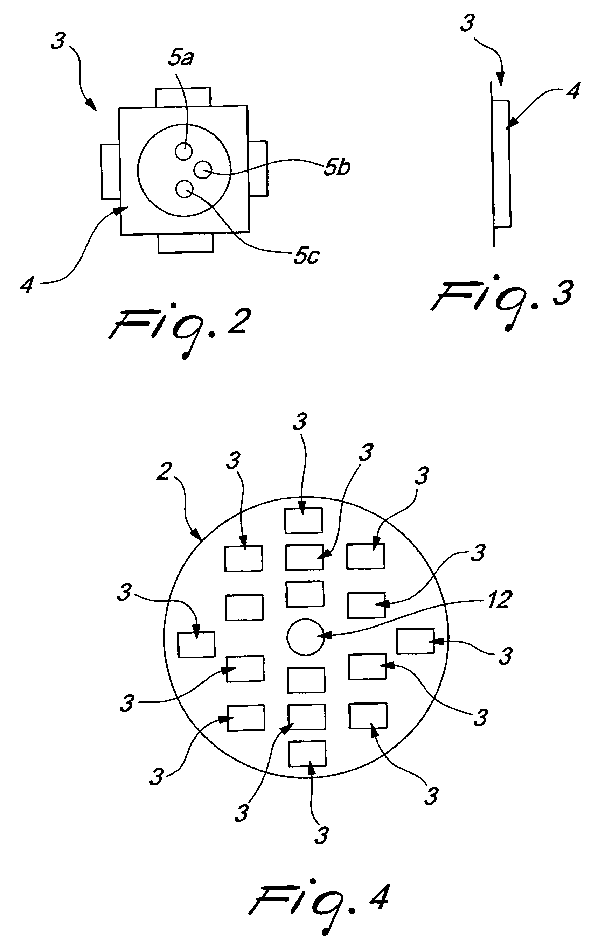

In particular, each one of said light sources with modulatable or adjustable color tint 3 can be constituted advantageously by an electronic component of a known type, termed "triple-junction LED", which is shown schematically in detail in Figures 2 and 3.

Said triple-junction LED comprises a header 4 with which three separate light emitters 5a, 5b and 5c are associated, each emitter being set to a preset wavelength, whose intensity can be modulated by means of an electronic control.

The wavelengths of the three emitters are conveniently chosen so that they correspond exactly to three complementary colors (for example yellow, magenta and cyan, or red, green and blue).

In this manner, by varying the intensity of each one of the three light emitters 5a, 5b and 5c substantially continuously, it is possible to achieve the emission of light of the chosen color tint.

For example, triple-junction LEDs with low-cost driving systems have 1024 color tints; with other more expensive and advanced driving systems it is possible, in theory, to use triple-junction LEDs so as to obtain up to almost 3 billion different color tints.

Currently commercially available triple-junction LEDs are used to produce sports, television and/or advertising billboards for broadcasting and displaying video images, acting like a computer monitor.

A protection device 6 is associable with the plate 2 at the front; said device is for example a transparent or semitransparent screen, which is suitable to protect the light sources 3 against humidity, dust and dirt and at the same time provide uniform diffusion of the light emitted by the light sources.

Moreover, the plate 2 can be associated in a rear region with, and/or accommodated within, a complementarily shaped compartment 7 formed in a box-like body 8 that is advantageously disk-shaped.

The box-like body 8, which in turn can be associated at the rear for example with a wall, is suitable to contain electronic control devices 9 with logic units for the light sources 3, particularly at least a first electronic controller, designated by the reference numeral 9a, which is connected electrically to one or more connectors 13.

One or more electronic inputs, generally designated by the reference numeral 10, are connected to the electronic controllers 9, so as to allow to control and manage the lighting device 1 according to a chosen color therapy program.

It is also possible to provide an electronic management that comprises at least two separate controllers: a first controller, again designated by the reference numeral 9a for the sake of simplicity, which is accommodated in the compartment 7, for interfacing with external control devices, such as for example a keypad or a remote control, and a second controller 9b, which is contained in a separate hermetic container and is suitable to control the light sources 3.

Such first and second controllers are mutually connected, for example by way of serial communications ports, such as the one designated by the reference numeral 11.

Moreover, the lighting device 1 can comprise automatic activation means, such as a presence and/or motion detection sensor, designated by the reference numeral 12 in Figures 1 and 4.

The motion sensor 12, when a person approaches or enters the sanitary fixture, can control for example the activation of the lighting device 1 and/or of the other electronic devices; likewise, it can control their deactivation when the user exits or after a preset period of time.

The motion sensor 12 is in fact interfaced with the first and/or second controllers, advantageously by means of the interposition of an electronic signal amplification circuit, which is not shown in Figure 1.

It is thus possible, for example, to program the automatic lighting in white of the lighting device 1 in case of correct operation of the sanitary fixture or in red in case of malfunction or any danger for the user's safety.

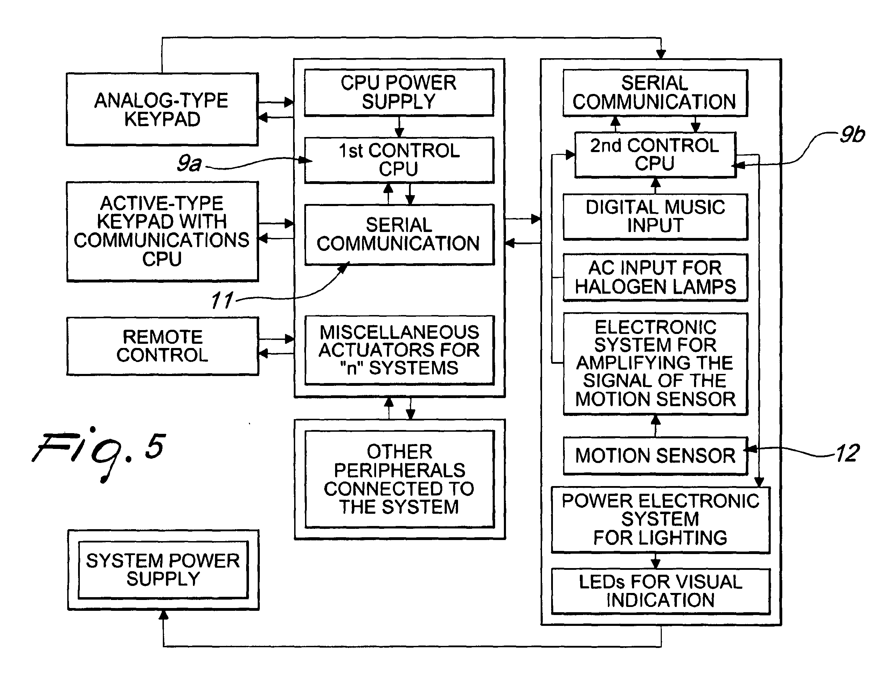

Figure 5 illustrates a possible schematic block diagram of the electronic circuit of the lighting device 1.

With reference to Figure 5, operation during power-on is therefore as follows: the motion sensor 12 detects the presence of the user and emits a signal that is amplified and sent to the second electronic controller 9b (2^{nd} control CPU).

By means of the serial communications ports, the signal is transmitted to the first controller 9a (1^{st} control CPU).

Once correct operation has been verified by way of autodiagnostic or diagnostic means of a known type, the external control devices (analog keypad, of the active type or remote control) warn the user of the clearance to use the fixture.

At the same time, the second controller sends a control signal to switch on the light sources 3 in white (or in any other color that corresponds to a message of correct operation).

During the operation of the lighting device 1, its management is entrusted to the first controller according to a preset color cycle or to other programming chosen by the user.

The sending of a preset electrical signal to the light sources with modulatable color tint 3 entails their lighting with preset intensities of said emitters 5a, 5b and 5c, and therefore the emission of light of a chosen color tint.

The light, which is per se of a uniform tint, is diffused in passing through the protection device 6, which can be fully transparent, so that the outer surface is therefore uniformly lit according to the chosen color tint.

It has thus been found that the invention has achieved the intended aim and objects, a lighting device having been devised which allows to use color therapy, in the field of sanitary fixtures, in a manner that is effective and adequate for the purposes of said therapy.

It is in fact possible to illuminate the surface of the device according to a chosen color tint, chosen within a very wide range of tints.

The light is also distributed entirely uniformly along said surface, and it is possible to vary the color tint manually or automatically according to a chosen color sequence.

The color tint variation, moreover, occurs uniformly in every point of the surface, if required also with a color intensity that is always constant.

The invention is of course susceptible of numerous modifications and variations, all of which are within the scope of the appended claims.

Thus, for example, it is possible to provide a lighting device that operates fully independently of the first controller and can therefore be activated directly by means of an analog keypad or a remote control.

It is also possible to select and store a chosen color or to program a customized color cycle.

It is also possible, by using the provided inputs and the first electronic controller, to interface the device with systems for synchronizing the color with music.

The invention can further be used to illuminate optical fibers uniformly, so as to transmit the light to remote regions of the sanitary fixture.

It is also possible to connect the first or second electronic controllers 9a, 9b to control means such as one or more actuators, such as electric valves, pumps, or other mechanisms for the control of the sanitary fixture, so as to control them autonomously or according to the signals sent to the lighting device.

Likewise, it is possible to connect to the controllers of the lighting device one or more peripherals, such as for example devices for listening to music, a telephone or a television set.

The materials used, as well as the dimensions that constitute the individual components of the invention, may of course be more pertinent according to specific requirements.

The various means for performing certain different functions need not certainly coexist only in the illustrated embodiment but can be present per se in many embodiments, including ones that are not illustrated.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A lighting device for sanitary fixtures, comprising a plurality of light sources (3) of modulatable color tint, which are arranged substantially uniformly on a support (2) and can be activated by first (9a) and second (9b) electronic controllers according to a chosen color therapy program, said first electronic controller (9a) comprising a first logic unit and being connectable to external control devices, **characterized by** comprising proximate to said light sources (3) with modulatable color tint, an automatic activation means (12) constituted by a presence and/or motion sensor for activating at least the lighting device (1) so as to provide a selective emission of at least one preset signaling color for the user, and said first electronic controller (9a) comprising a diagnostic means for verifying correct operation of sanitary fixtures and for sending to said second controller (9b) a preset signal determining the signaling color with which the lighting device (1) lights up.

2. The lighting device according to claim 1, **characterized in that** each one of said light sources (3) with modulatable color tint can be activated by said electronic controllers (9a,9b) in order to emit a chosen color tint, selected among a plurality of different color tints.

3. The lighting device according to claims 1 and 2, **characterized in that** said light sources (3) with modulatable color tint are constituted by triple junction LEDs.

4. The lighting device according to claims 1 and 3, **characterized in that** said light sources (3) with modulatable color tint are activated by the same control signal of said electronic controllers (9a,9b) so as to emit simultaneously the same color tint.

5. The lighting device according to one or more of the preceding claims, **characterized in that** said external control devices comprise a keypad and/or a remote control, for the activation and/or adjustment at least of the lighting device (1).

6. The lighting device according to claims 5, **characterized in that** said second electronic controller (9b) comprises a second logic unit that receives a signal from said presence and/or motion sensor (12), for activation of the lighting device (1) when a user is present.

7. The lighting device according to claim 6, **characterized in that** said second electronic controller (9b), which comprises a second logic unit receives a signal from said presence and/or motion sensor (12) in order to control said first controller (9a) so as to achieve activation of said keypad and/or remote control.

8. The lighting device according to claim 7, **characterized in that** said first electronic controller (9a) is accommodated in a compartment (7) formed in a box-like body (8) for interfacing with said external control devices, and said second electronic controller (9b) is contained in a separate hermetic container, said first and second electronic controllers (9a, 9b) being mutually interconnected by way of serial communication ports (11).

9. The lighting device according to one or more of the preceding claims, **characterized in that** said light sources (3) with modulatable color tint are associated with, or arranged proximate to, the ends of respective optical fibers in order to transmit the light to remote regions.

10. The lighting device according to one or more of the preceding claims, **characterized in that** it is interfacable with systems for synchronizing the colour with music.

11. The lighting device according to one or more of the preceding claims, **characterized in that** said first (9a) or said second (9b) electronic controller is connectable to control means, such as electric valves, pumps or other mechanisms for the control of the sanitary fixtures, so as to control them according to signals sent to the lighting device, and possibly to one or more peripherals, such as a telephone and a television set.

## Patentansprüche

1. Eine Beleuchtungsvorrichtung für sanitäre Einrichtungen, die eine Vielzahl von Lichtquellen (3) mit modulierbarem Farbton umfasst, welche im Wesentlichen gleichmäßig auf einem Träger (2) angeordnet sind und von ersten (9a) und zweiten (9b) elektronischen Steuerungen entsprechend einem ausgewählten Farbtherapie-Programm aktiviert werden können, wobei die erste elektronische Steuerung (9a) eine erste Logikeinheit umfasst und mit externen Steuerungsvorrichtungen verbunden werden kann, **dadurch gekennzeichnet, dass** sie, nahe den Lichtquellen (3) mit modulierbarem Farbton, ein automatisches Aktivierungsmittel (12) umfasst, das aus einem Anwesenheits- und/oder Bewegungssensor zur Aktivierung zumindest der Beleuchtungsvorrichtung (1) besteht, um eine selektive Ausstrahlung mindestens einer voreingestellten Signalfarbe für den Benutzer bereitzustellen, und wobei die erste elektronische Steuerung (9a) ein Diagnosemittel umfasst, um den korrekten Betrieb der sanitären Einrichtung zu überprüfen und um an die zweite Steuerung (9b) ein voreingestelltes Signal zu senden, welches die Signalfarbe bestimmt, mit der die Beleuchtungsvorrichtung (1) aufleuchtet.

2. Die Beleuchtungsvorrichtung gemäß Anspruch 1, **dadurch gekennzeichnet, dass** jede der Lichtquellen (3) mit modulierbarem Farbton von den elektronischen Steuerungen (9a, 9b) aktiviert werden kann, um einen ausgewählten Farbton, gewählt aus einer Vielzahl verschiedener Farbtöne, zu emittieren.

3. Die Beleuchtungsvorrichtung gemäß, Anspruch 1 und 2, **dadurch gekennzeichnet, dass** die Lichtquellen (3) mit modulierbarem Farbton aus Dreifachverbindungs-LEDs bestehen.

4. Die Beleuchtungsvorrichtung gemäß Anspruch 1 und 3, **dadurch gekennzeichnet, dass** die Lichtquellen (3) mit modulierbarem Farbton von demselben Steuersignal der elektronischen Steuerungen (9a, 9b) aktiviert werden, um gleichzeitig denselben Farbton.zu emittieren.

5. Die Beleuchtungsvorrichtung gemäß einem oder mehreren der obigen Anspruche, **dadurch gekennzeichnet, dass** die externen Steuerungsvorrichtungen eine Tastatur und/oder eine Fernbedienung zur Aktivierung und/oder Einstellung zumindest der Beleuchtungsvorrichtung (1) umfassen.

6. Die Beleuchtungsvorrichtung gemäß Anspruch 5, **dadurch gekennzeichnet, dass** die zweite elektronische Steuerung (9b) eine zweite Logikeinheit umfasst, die zur Aktivierung der Beleuchtungsvorrichtung (1), wenn ein Benutzer anwesend ist, ein Signal von dem Anwesenheits- und/oder Bewegungssensor (12) empfängt.

7. Die Beleuchtungsvorrichtung gemäß Anspruch 6, **dadurch gekennzeichnet, dass** die zweite elektronische Steuerung (9b), die eine zweite Logikeinheit umfasst, ein Signal von dem Anwesenheits- und/oder Bewegungssensor (12) empfängt, um die erste Steuerung (9a) zu steuern, um eine Aktivierung der Tastatur und/oder Fernbedienung zu erzielen.

8. Die Beleuchtungsvorrichtung gemäß Anspruch 7, **dadurch gekennzeichnet, dass** die erste elektronische Steuerung (9a) in einem Fach (7) untergebracht ist, welches in einem gehäuseartigen Kasten (8) geformt ist, zur Verbindung mit den externen Steuerungsvorrichtungen, und die zweite elektronische Steuerung (9b) in einem separaten luftdichten Behälter enthalten ist, wobei die erste und die zweite elektronische Steuerung (9a, 9b) miteinander durch serielle Übertragungsanschlüsse (11) verbunden sind.

9. Die Beleuchtungsvorrichtung gemäß einem oder mehreren der obigen Ansprüche, **dadurch gekennzeichnet, dass** die Lichtquellen (3) mit modulierbarem Farbton mit den Enden entsprechender Glasfasern verknüpft oder in deren Nähe angeordnet sind, um das Licht an entfernte Bereiche zu übertragen.

10. Die Beleuchtungsvorrichtung gemäß einem oder mehreren der obigen Ansprüche, **dadurch gekennzeichnet, dass** sie mit Systemen zur Synchronisierung der Farbe mit Musik verbunden werden kann.

11. Die Beleuchtungsvorrichtung gemäß einem oder mehreren der obigen Ansprüche, **dadurch gekennzeichnet, dass** die erste (9a) oder die zweite (9b) elektronische Steuerung an Steuerungsmittel, wie z. B. elektrische Ventile, Pumpen oder andere Mechanismen zur Steuerung der sanitären Einrichtungen, verbunden werden können, um sie entsprechend Signalen, welche an die Beleuchtungsvorrichtung, und möglicherweise an ein oder mehrere Peripheriegeräte, wie z. B. ein Telefon und einen Fernseher, gesendet werden, zu steuern.

## Revendications

1. Dispositif d'éclairage pour des installations sanitaires, comprenant une pluralité de sources lumineuses (3) à teinte chromatique modulable, qui sont agencées sensiblement uniformément sur un support (2) et peuvent être actionnées par un premier (9a) et un second (9b) dispositifs de commande électroniques selon un programme de chromothérapie choisi, ledit premier dispositif de commande électronique (9a) comprenant une première unité logique et pouvant être reliée à des dispositifs de commande externes,
**caractérisé en ce qu'**il comprend à proximité desdites sources lumineuses (3) à teinte chromatique modulable, des moyens d'actionnement automatiques (12) constitués par un capteur de présence et/ou de mouvement pour actionner au moins le dispositif d'éclairage (1) de façon à fournir une émission sélective d'au moins une couleur de signalisation prédéterminée pour l'utilisateur et ledit premier dispositif de commande électronique (9a) comprenant des moyens de diagnostic pour vérifier le fonctionnement correct des installations sanitaires et pour envoyer audit second dispositif de commande (9b) un signal prédéterminé déterminant la couleur de signalisation avec laquelle le dispositif d'éclairage (1) s'allume.

2. Dispositif d'éclairage selon la revendication 1,
**caractérisé en ce que** chacune desdites sources lumineuses (3) à teinte chromatique modulable peut être actionnée par lesdits dispositifs de commande électroniques (9a, 9b) de façon à émettre une teinte chromatique choisie, sélectionnée parmi une pluralité de différentes teintes chromatiques.

3. Dispositif d'éclairage selon les revendications 1 et 2;
**caractérisé en ce que** lesdites sources lumineuses (3) à teinte chromatique modulable sont constituées par des diodes électroluminescentes à triple jonction.

4. Dispositif d'éclairage selon les revendications 1 et 3,
**caractérisé en ce que** lesdites sources lumineuses (3) à teinte chromatique modulable sont actionnées par le même signal de commande desdits dispositifs de commande électroniques (9a, 9b), de façon à émettre simultanément la même teinte chromatique.

5. Dispositif d'éclairage selon une ou plusieurs des revendications précédentes,
**caractérisé en ce que** lesdits dispositifs de commande externes comprennent un clavier et/ou une commande à distance, pour actionner et/ou régler au moins le dispositif d'éclairage (1).

6. Dispositif d'éclairage selon la revendication 5,
**caractérisé en ce que** ledit second dispositif de commande électronique (9b) comprend une seconde unité logique qui reçoit un signal dudit capteur de présence et/ou de mouvement (12), pour l'actionnement du dispositif d'éclairage (1) quand un utilisateur est présent.

7. Dispositif d'éclairage selon la revendication 6,
**caractérisé en ce que** ledit second dispositif de commande électronique (9b), qui comprend une seconde unité logique, reçoit un signal dudit capteur de présence et/ou de mouvement (12) de façon à commander ledit premier dispositif de commande (9a) pour obtenir l'actionnement dudit clavier et/ou de ladite commande à distance.

8. Dispositif d'éclairage selon la revendication 7,
**caractérisé en ce que** ledit premier dispositif de commande électronique (9a) est logé dans un compartiment (7) formé dans un corps en forme de boite (8) pour communiquer avec lesdits dispositifs de commande externes, et ledit second dispositif de commande électronique (9b) est contenu dans un boîtier hermétique séparé, lesdits premier et second dispositifs de commande électroniques (9a, 9b) étant mutuellement reliés par l'intermédiaire de ports de communication en série (11).

9. Dispositif d'éclairage selon une ou plusieurs des revendications précédentes,
**caractérisé en ce que** lesdites sources lumineuses (3) à teinte chromatique modulable sont associées aux extrémités ou agencées à proximité des extrémités de fibres optiques respectives de façon à transmettre la lumière vers des zones éloignées.

10. Dispositif d'éclairage selon une ou plusieurs des revendications précédentes,
**caractérisé en ce qu'**il peut communiquer avec des systèmes pour synchroniser la couleur avec la musique.

11. Dispositif d'éclairage selon une ou plusieurs des revendications précédentes,
**caractérisé en ce que** ledit premier dispositif de commande électronique (9a) ou ledit second dispositif de commande électronique (9b) peut être relié à des moyens de commande, tels que des électrovalves, des pompes ou autres mécanismes pour la commande des installations sanitaires, de façon à les commander selon des signaux envoyés au dispositif d'éclairage, et éventuellement à un ou plusieurs périphériques, comme un téléphone et un téléviseur.
